**Europäisches Patentamt**

(19) **European Patent Office**

**Office européen des brevets**

(11) Veröffentlichungsnummer : **0 154 123**
**B1**

(12) **EUROPÄISCHE PATENTSCHRIFT**

(45) Veröffentlichungstag der Patentschrift :
22.02.89

(21) Anmeldenummer : 85100352.5

(22) Anmeldetag : 15.01.85

(51) Int. Cl.⁴ : **C 22 C 5/02**, C 22 C 5/04,
A 61 K 6/04

(54) Edelmetallegierungen für Dentalanwendungen.

(30) Priorität : 24.02.84 DE 3406712

(43) Veröffentlichungstag der Anmeldung :
11.09.85 Patentblatt 85/37

(45) Bekanntmachung des Hinweises auf die Patenterteilung : 22.02.89 Patentblatt 89/08

(84) Benannte Vertragsstaaten :
AT BE CH DE FR GB IT LI LU NL SE

(56) Entgegenhaltungen :
DE--A-- 2 814 943
DE--C-- 2 813 813
GB--A-- 2 048 939
US--A-- 4 419 325

(73) Patentinhaber : **Degussa Aktiengesellschaft**
**Weissfrauenstrasse 9**
**D-6000 Frankfurt am Main 1 (DE)**

(72) Erfinder : **Nawaz, M. H. A., Dr.**
**Hinter den Gärten 6**
**D-7531 Neulingen (DE)**

**Beschreibung**

Die Erfindung betrifft Edelmetallegierungen für Dentalanwendungen, insbesondere zum Aufbrennen von keramischen Massen, aus 20 bis 65 % Gold, 25 bis 65 % Palladium, 0 bis 7 % Gallium, 0,2 bis 11 % Indium und/oder Zinn, 0 bis 2 % Kupfer, 0,05 bis 1 % Ruthenium, Rhenium und/oder Iridium, 0 bis 1 % Vanadin und 0 bis 1 % Eisen.

Die üblichen Dentallegierungen mit einem Goldgehalt zwischen 70 und 85 Gewichtsprozent sind zur Herstellung von Zahnprothesen gut geeignet (z. B. DE-PS 15 33 233). Sie sind in der Mundhöhle korrosionsbeständig und lassen sich problemlos verarbeiten. So werden Dentalprothesen beispielsweise im Wachsausschmelzverfahren hergestellt.

In jüngerer Zeit werden auch Legierungen verwendet, in denen ein Teil des Goldgehaltes durch Palladium ersetzt ist. Um die guten Schmelz- und Gießeigenschaften der hochgoldhaltigen Legierungen beizubehalten, wird zusätzlich in einigen dieser goldreduzierten Legierungen der Silbergehalt erhöht (z. B. DE-PS 24 40 425). Ein ästhetischer Nachteil solcher Legierungen ist jedoch, daß sich die Dentalkeramik während des Aufbrennprozesses durch das Silber verfärben kann.

Der genaue Mechanismus, welcher eine solche Verfärbung verursacht, ist noch nicht bekannt, Legierungen ohne Silber zeigen dieses Verhalten jedoch nicht.

Die bekannten silberfreien Gold-Palladium-Legierungen (z. B. DE-PS 28 13 813) besitzen dagegen eine hohe Liquidustemperatur, dis das Schmelzen in den üblichen zahntechnischen Geräten erschwert. Solche Legierungen zeigen außerdem nicht das Formfüllungsvermögen und die Fließeigenschaften der hochgoldhaltigen oder der goldreduzierten, aber silberhaltigen Legierungen, was zu Fehlern im Guß und zu Problemen bei der Verblendung führen kann. Zum Aufbrennen von Keramik geeignete Legierungen müssen zusätzlich in ihrem Wärmeausdehnungsverhalten dem der keramischen zu hohe Massen ähnlich sein, damit in der Keramikschicht Spannungen vermieden werden, welche zu Rissen in der Keramik führen können. Üblicherweise ist der Ausdehnungskoeffizient von goldreduzierten, silberfreien Legierungen niedriger als der der verwendeten Keramik. Dies führt oft zu Rissen oder Sprüngen in der Keramik. Zur Verbesserung der Wirtschaftlichkeit ist es notwendig, Gußabfälle wiederzuverwenden. Die Legierungen müssen daher mehrmals geschmolzen und vergossen werden können, ohne daß sich ihre Eigenschaften verändern, was zu Gußbrüchen und zu Abplatzungen der Keramik führen kann.

Aus der US-PS 4 419 325 sind Palladiumlegierungen für Dentalanwendungen bekannt, die neben 35 bis 85 % Palladium 5 bis 15 % Gallium, 0,1 bis 0,5 % Ruthenium oder Rhenium, 0 bis 50 % Gold, 0 bis 12 % Kupfer, 0 bis 5 % Aluminium und 0 bis 13 % Kobalt enthalten. Diese Legierungen besitzen ein relativ hohes Schmelzintervall und zeigen kein optimales Haftungsvermögen auf den üblichen Dentalkeramiken. Außerdem müssen sie unter Vakuum oder Inertgas erschmolzen werden. In der DE-OS 29 44 755 werden zahlreiche zahntechnische Haftbrennlegierungen mit ca. 32 bis 63 % Gold und 29 bis 58 % Palladium beschrieben, die außerdem noch 0,5 bis 10,55 % Indium enthalten und denen bis zu einigen Prozenten Gallium, Zinn, Kupfer, Nickel, Aluminium, Titan und Silber zugesetzt sein können. Diese Legierungen verändern jedoch ihre Eigenschaften bei mehrfachem Umschmelzen und ergeben dann keine dichten und lunkerfreien Güsse mehr.

Nach dem Verblenden der Gußstücke mit Keramik ist es üblich, verschiedene Teile einer Dentalbrücke im Ofen zu löten. Man benutzt hierzu Flußmittel, die die Keramik nicht angreifen. Mit den Legierungen nach der DE-OS 29 44 755 ist es nicht möglich, duktile zuverlässige Lötungen mit kadmiumfreien Loten zu bekommen. Die Lötverbindung bricht ohne plastische Verformung an der Verbindungsfläche zwischen Lot und Legierung ab.

Es war daher Aufgabe der vorliegenden Erfindung, Edelmetallegierungen für Dentalanwendungen, insbesondere zum Aufbrennen von dentalkeramischen Massen zu schaffen, aus 20 bis 65 % Gold, 25 bis 65 % Palladium, 0 bis 7 % Gallium, 0,2 bis 11 % Indium und/oder Zinn, 0 bis 2 % Kupfer, 0,05 bis 1 % einem oder mehreren der Elemente Ruthenium, Iridium und Rhenium, 0 bis 1 % Vanadin und 0 bis 1 % Eisen, die niedrigschmelzend sind, um gut verarbeitbar zu sein, trotzdem eine hohe Solidustemperatur besitzen, damit sie während des Keramikaufbrennprozesses nicht verformt werden, und nicht zu Verfärbungen in der Keramik führen. Außerdem sollten sie ohne Eigenschaftsänderungen mehrfach an Luft schmelz- und giessbar sein und mit kadmiumfreien Loten dauerhafte Lötungen ermöglichen.

Diese Aufgabe wurde erfindungsgemäß dadurch gelöst, daß sie zusätzlich 0,5 bis 15 % Kobalt enthalten, wobei der Gesamtgehalt an Unedelmetallen über 5 % liegen muß.

Besonders geeignete Legierungen enthalten 50 bis 65 % Gold, 30 bis 40 % Palladium, 4 bis 10 % Indium, 0 bis 2 % Zinn, 1 bis 2 % Gallium, 0,05 bis 1 % Ruthenium, Iridium und/oder Rhenium, und 1 bis 5 % Kobalt.

Vorzugsweise verwendet man Legierungen mit 60 bis 65 % Gold, 30 bis 35 % Palladium, 4 bis 6 % Indium, 0 bis 2 % Zinn, 1 bis 2 % Gallium, 0,05 bis 0,3 % Ruthenium, Iridium und/oder Rhenium und 1 bis 3 % Kobalt.

Die erfindungsgemäßen Legierungen verfärben die handelsüblichen Keramiken in keiner Weise. Der Kobaltgehalt hat sich überraschenderweise bei diesen Aufbrennlegierungen als besonders geeignet erwiesen, die Eigenschaften zu verbessern bzw. die bisherigen Mängel zu beseitigen. Die Legierungen sind besonders niedrigschmelzend und lassen sich problemlos in handelsüblichen Gießgeräten, wie z. B.

2

widerstandsbeheizten Öfen, oder mit der Propan-Sauerstoff-Flamme schmelzen. Ihre Fließ- und Formfüllungseigenschaften ergeben dichte und lunkerfreie Güsse, auch nach mehrfachem Umschmelzen. Ihre Solidustemperatur liegt ausreichend hoch, so daß eine Verformung während des Aufbrennprozesses ausgeschlossen ist. Es hat sich außerdem herausgestellt, daß Lötungen mit diesen Legierungen hohe Festigkeit und zähes Bruchverhalten zeigen.

Nach mehrfachem Schmelzen und Gießen dieser Legierungen wurden weder Gußbrüche noch Abplatzungen festgestellt. Auch konnten die Legierungen problemlos weiterverarbeitet werden. Der thermische Ausdehnungskoeffizient stimmt mit dem der üblichen Dentalkeramiken optimal überein, sodaß auch nach mehreren Monaten weder Risse noch Sprünge in der Keramik feststellbar sind. Auch die Haftung ist sehr gut und den meisten bekannten Aufbrennlegierungen überlegen.

Die Legierungen eignen sich aufgrund ihres guten Giessverhaltens auch als Gußlegierungen für herausnehmbaren Zahnersatz und als Gerüste für Kunststoffverblendungen.

Gute Eigenschaften zeigen auch goldärmere Legierungen. Bewährt haben sich Legierungen mit 50 bis 55 % Gold, 35 bis 40 % Palladium, 6 bis 10 % Indium, 0 bis 1 % Zinn, 1 bis 2 % Gallium, 0,05 bis 1 % Ruthenium, Iridium und/oder Rhenium, 1,5 bis 12,5 % Kobalt, 0 bis 1 % Vanadium und 0 bis 1 % Eisen, bzw. Legierungen mit 20 bis 50 % Gold, 40 bis 60 % Palladium, 0,2 bis 8 % Indium, 0,2 bis 6 % Zinn, 0 bis 2 % Kupfer, 1 bis 5 % Gallium, 0,05 bis 1 % Ruthenium, Iridium und Rhenium und 2 bis 12 % Kobalt.

Die nachstehende Tabelle zeigt die Eigenschaften einiger erfindungsgemäßen Legierungen :

(Siehe Tabelle Seite 4 f.)

Zusammensetzung (Massengehalt in %)

| Nr. | Au | Pd | In | Sn | Co | V | Fe | Ga | Cu | Ru | Re | Ir | Schmelzintervall °C | Härte HV 5 | | |
|---|---|---|---|---|---|---|---|---|---|---|---|---|---|---|---|---|
| | | | | | | | | | | | | | | g | w | a |
| 1 | 61,6 | 30,0 | 5,0 | 0,5 | 1,5 | | | 1,2 | | | | 0,2 | 1255-1165 | 230 | 190 | 230 |
| 2 | 61,6 | 30,0 | 4,0 | | 2,7 | | | 1,5 | | | | 0,2 | 1245-1145 | 220 | 190 | 245 |
| 3 | 55,0 | 35,0 | 6,5 | 0,2 | 2,1 | | | 1,0 | | | | 0,2 | 1275-1150 | 232 | 180 | 232 |
| 4 | 55,0 | 35,0 | 6,5 | | 2,1 | | | 1,2 | | | | 0,2 | 1270-1150 | 232 | 201 | 234 |
| 5 | 53,5 | 36,0 | 5,6 | | 3,2 | | | 1,5 | | | 0,2 | | 1265-1195 | 230 | 215 | 230 |
| 6 | 52,5 | 35,1 | 8,0 | | 2,7 | | | 1,5 | | | 0,2 | | 1220-1145 | 305 | 270 | 305 |
| 7 | 52,5 | 35,1 | 8,0 | | 2,2 | 0,5 | | 1,5 | | | 0,2 | | 1220-1145 | 277 | 244 | 283 |
| 8 | 52,5 | 33,1 | | 2,0 | 12,2 | | | | | 0,2 | | | 1230-1150 | 217 | 177 | 247 |
| 9 | 52,5 | 33,1 | | 2,0 | 11,7 | | | 0,5 | | 0,2 | | | 1220-1140 | 265 | 232 | 321 |
| 10 | 52,5 | 35,1 | 8,0 | 0,5 | 2,2 | | | 1,5 | | 0,2 | | | 1220-1150 | 293 | 249 | 293 |
| 11 | 52,5 | 35,1 | 7,2 | 1,3 | 2,2 | | | 1,5 | | 0,2 | | | 1220-1150 | 286 | 280 | 303 |
| 12 | 52,5 | 35,1 | 8,0 | 0,5 | 2,2 | | | 1,5 | | | 0,2 | | 1220-1145 | 285 | 255 | 305 |
| 13 | 51,9 | 37,0 | 6,5 | 0,5 | 2,7 | | | 1,2 | | | 0,2 | | 1260-1190 | 245 | 220 | 245 |
| 14 | 51,1 | 37,0 | 7,0 | 0,5 | 2,2 | | 0,5 | 1,5 | | | 0,2 | | 1230-1150 | 255 | 255 | 270 |
| 15 | 51,1 | 37,0 | 7,0 | 0,5 | 2,2 | 0,5 | | 1,5 | | | 0,2 | | 1220-1150 | 230 | 215 | 230 |
| 16 | 51,1 | 36,5 | 8,0 | | 2,7 | | | 1,5 | | | | 0,2 | 1245-1145 | 260 | 245 | 270 |
| 17 | 51,1 | 36,5 | 7,5 | | 3,2 | | | 1,5 | | | | 0,2 | 1220-1145 | 270 | 255 | 270 |
| 18 | 51,1 | 37,0 | 7,0 | | 3,2 | | | 1,5 | | | | 0,2 | 1235-1145 | 270 | 255 | 270 |
| 19 | 51,1 | 37,0 | 7,0 | 0,5 | 2,7 | | | 1,5 | | | | 0,2 | 1235-1145 | 260 | 245 | 270 |
| 20 | 45,0 | 41,7 | 5,0 | | 4,8 | | | 3,2 | | 0,3 | | | 1210-1140 | 285 | 255 | 360 |
| 21 | 45,0 | 41,7 | 4,2 | 0,8 | 4,8 | | | 3,2 | | 0,3 | | | 1210-1140 | 285 | 255 | 340 |
| 22 | 40,0 | 46,6 | 5,0 | 0,8 | 4,1 | | | 1,5 | 1,8 | 0,2 | | | 1270-1140 | 201 | 175 | 210 |
| 23 | 35,0 | 48,2 | 4,0 | 2,0 | 7,0 | | | 3,5 | | 0,3 | | | 1200-1150 | 321 | 293 | 321 |
| 24 | 20,0 | 59,6 | | 6,0 | 10,0 | | | 4,0 | | 0,4 | | | 1210-1150 | 372 | 314 | 412 |

g = Gußhärte    w = weichgeglüht    a = maximal ausgehärtet

EP 0 154 123 B1

## EP 0 154 123 B1

**Patentansprüche**

1. Edelmetallegierungen für Dentalanwendungen, insbesondere zum Aufbrennen von dentalkeramischen Massen, bestehend aus 20 bis 65 % Gold, 25 bis 65 % Palladium, 0 bis 7 % Gallium, 0,2 bis 11 % Indium und/oder Zinn, 0 bis 2 % Kupfer, 0,05 bis 1 % einem oder mehreren der Elemente Ruthenium, Iridium und Rhenium, 0 bis 1 % Vanadin, 0 bis 1 % Eisen und 0,5 bis 15 % Kobalt wobei der Gehalt an Unedelmetallen über 5 % liegen muß.

2. Edelmetallegierungen nach Anspruch 1, dadurch gekennzeichnet, daß sie 50 bis 65 % Gold, 30 bis 40 % Palladium, 4 bis 10 % Indium, 0 bis 2 % Zinn, 1 bis 2 % Gallium, 0,05 bis 1 % eines oder mehrerer der Elemente Ruthenium, Rhenium und Iridium und 1 bis 5 % Kobalt enthalten.

3. Edelmetallegierungen nach Anspruch 1 und 2, dadurch gekennzeichnet, daß sie 60 bis 65 % Gold, 30 bis 35 % Palladium, 4,5 bis 6 % Indium, 0 bis 2 % Zinn, 1 bis 2 % Gallium, 0,05 bis 0,3 % eines oder mehrerer der Elemente Ruthenium, Iridium, Rhenium und 1 bis 3 % Kobalt enthalten.

4. Edelmetallegierungen nach Anspruch 1, dadurch gekennzeichnet, daß sie 50 bis 55 % Gold, 35 bis 40 % Palladium, 6 bis 10 % Indium, 0 bis 1 % Zinn, 1 bis 2 % Gallium, 0,05 bis 1 % Ruthenium, Rhenium und/oder Iridium, 1,5 bis 12,5 % Kobalt, 0 bis 1 % Vanadin und 0 bis 1 % Eisen enthalten.

5. Edelmetallegierungen nach Anspruch 1, dadurch gekennzeichnet, daß sie 20 bis 50 % Gold, 40 bis 60 % Palladium, 0,2 bis 8 % Indium, 0,2 bis 6 % Zinn, 0 bis 2 % Kupfer, 1 bis 5 % Gallium, 0,05 bis 1 % eines oder mehrerer der Elemente Ruthenium, Iridium und Rhenium und 2 bis 12 % Kobalt enthalten.

**Claims**

1. Noble metal alloys for dental uses, in particular for fusing on dental ceramic compositions, which consist of 20 to 65 % of gold, 25 to 65 % of palladium, 0 to 7 % of gallium, 0.2 to 11 % of indium and/or tin, 0 to 2 % of copper, 0.05 to 1 % of one or more of the elements ruthenium, iridium and rhenium, 0 to 1 % of vanadium, 0 to 1 % of iron and 0.5 to 15 % of cobalt, the non-noble metal content needing to be above 5 %.

2. Noble metal alloys according to claim 1, characterized in that they contain 50 to 65 % of gold, 30 to 40 % of palladium, 4 to 10 % of indium, 0 to 2 % of tin, 1 to 2 % of gallium, 0.05 to 1 % of one or more of the elements ruthenium, rhenium and iridium and 1 to 5 % of cobalt.

3. Noble metal alloys according to claim 1 and 2, characterized in that they contain 60 to 65 % of gold, 30 to 35 % of palladium, 4.5 to 6 % of indium, 0 to 2 % of tin, 1 to 2 % of gallium, 0.05 to 0.3 % of one or more of the elements ruthenium, iridium, rhenium and 1 to 3 % of cobalt.

4. Noble metal alloys according to claim 1, characterised in that they contain 50 to 55 % of gold, 35 to 40 % of palladium, 6 to 10 % of indium, 0 to 1 % of tin, 1 to 2 % of gallium, 0.05 to 1 % of ruthenium, rhenium and/or iridium, 1.5 to 12.5 % of cobalt, 0 to 1 % of vanadium and 0 to 1 % of iron.

5. Noble metal alloys according to claim 1, characterized in that they contain 20 to 50 % of gold, 40 to 60 % of palladium, 0.2 to 8 % of indium, 0.2 to 6 % of tin, 0 to 2 % of copper, 1 to 5 % of gallium, 0.05 to 1 % of one or more of the elements ruthenium, iridium and rhenium and 2 to 12 % of cobalt.

**Revendications**

1. Alliages en métal noble pour des utilisations dentaires, en particulier pour le chauffage de masses de céramique dentaire, comprenant 20 à 65 % d'or, 25 à 65 % de palladium, 0 à 7 % de gallium, 0,2 à 11 % d'indium et/ou d'étain, 0 à 2 % de cuivre, 0,05 à 1 % d'un ou plusieurs éléments choisis dans le groupe du ruthénium, de l'iridium et du rhénium, 0 à 1 % de vanadium, 0 à 1 % de fer et 0,5 à 15 % de cobalt pour lesquels la teneur en métaux non nobles doit se situer au-dessus de 5 %.

2. Alliages en métal noble selon la revendication 1, caractérisés en ce qu'ils contiennent de 50 à 65 % d'or, 30 à 40 % de palladium, de 40 à 10 % d'indium, de 0 à 2 % d'étain, de 1 à 2 % de gallium, de 0,05 à 1 % d'un ou plusieurs éléments choisis dans le groupe du ruthénium, de l'iridium et du rhénium, et de 1 à 5 % de cobalt.

3. Alliages en métal noble selon les revendications 1 et 2, caractérisés en ce qu'ils contiennent de 60 à 65 % d'or, de 30 à 35 % de palladium, de 4,5 à 6 % d'indium, de 0 à 2 % d'étain, de 1 à 2 % de gallium, de 0,05 à 0,3 % d'un ou plusieurs éléments choisis dans le groupe du ruthénium, de l'iridium et du rhénium, et de 1 à 3 % de cobalt.

4. Alliages en métal noble selon la revendication 1, caractérisés en ce qu'ils contiennent de 50 à 55 % d'or, de 35 à 40 % de palladium, 6 à 10 % d'indium, de 0 à 1 % d'étain, de 1 à 2 % de gallium, de 0,05 à 1 % de ruthénium, rhénium et/ou iridium, de 1,5 à 12,5 % de cobalt, de 0 à 1 % de vanadium et de 0 à 1 % de fer.

5. Alliages en métal noble selon la revendication 1, caractérisés en ce qu'ils contiennent de 20 à 50 % d'or, 40 à 60 % de palladium, 0,2 à 8 % d'indium, 0,2 à 6 % d'étain, 0 à 2 % de cuivre, 1 à 5 % de gallium, 0,05 à 1 % d'un ou plusieurs éléments choisis dans le groupe du ruthénium, iridium et rhénium, et de 2 à 12 % de cobalt.